# EUROPÄISCHE PATENTSCHRIFT

(11) **EP 0 514 367 B1**
(45) Veröffentlichungstag und Bekanntmachung des Hinweises auf die Patenterteilung: **07.08.1996**
(21) Anmeldenummer: 92890108.1
(22) Anmeldetag: 11.05.1992
(51) Int. Cl.: A61K 38/48, A61K 38/00, A61K 38/46

(54) **Arzneimittel enthaltend Protein C**
Protein C-containing drug
Médicament contenant de la protéine C

(30) Priorität: 14.05.1991 AT 991/91
(43) Veröffentlichungstag der Anmeldung: 19.11.1992
(73) Patentinhaber: IMMUNO Aktiengesellschaft, A-1221 Wien (AT)
(72) Erfinder: Eibl, Johann, Dr., A-1180 Wien (AT); Pichler, Ludwig, Prof. Dr., A-1040 Wien (AT); Schwarz, Hans Peter, Doz. Dr., A-1180 Wien (AT)
(74) Vertreter: Wolfram, Gustav, Dipl.-Ing.

(56) Entgegenhaltungen:
- EP-A- 0 318 201
- EP-A- 0 471 660
- WO-A-90/08556
- WO-A-90/12028
- WO-A-91/09960
- MEDLINE ABSTRACT, Number 82157904; CHU D.Z. et al.: " Purpura fulminans"
- The New England Journal of medicine, vol. 352, no.22, p. 1565-1568, 1991, Dreyfus et al;
- Seminars in Thrombosis and Hemastsis; vol. 16, no. 4, p. 299-309, Marlar et al;

## Beschreibung

Die Erfindung betrifft eine neue Verwendung von Protein C bzw. aktiviertem Protein C zur Herstellung eines Arzneimittels.

Protein C ist ein Vitamin-K-abhängiges Glycoprotein, das in der Leber synthetisiert wird und im Plasma in einer Konzentration von 4 µg/ml als inaktives Zymogen zirkuliert. Es wird an der Gefäßwandoberfläche (Endothel) durch den Thrombin-Thrombomodulinkomplex in die aktive Serinprotease (aktiviertes Protein C) übergeführt. Es ist bekannt, daß aktiviertes Protein C profibrinolytische Eigenschaften hat. Es wirkt auch antikoagulatorisch, weil es den Faktor Va, den Kofaktor für die Faktor Xa-induzierte Prothrombinaktivierung (Thrombinbildung) und den Faktor VIIIa, den Kofaktor für die Faktor IXa-induzierte Faktor X-Aktivierung, durch Proteolyse abbaut.

Die Aktivierung von Protein C in vivo stellt eine negative Feedback-Reaktion der Thrombingenerierung dar. Ein Verfahren zur Herstellung von aktiviertem Protein C in vitro wird z.B. in der EP-A - 0 416 890 beschrieben.

Es ist bekannt, daß aktiviertes Protein C im Zusammenhang mit Sepsis und septischem Schock vorteilhafte Wirkung zeigt.

Der septische Schock ist die systemische Reaktion auf die Freisetzung von Endotoxinen, Lipopolysaccharidbestandteilen der Zellwand gramnegativer Bakterien, in die Zirkulation. Der septische Schock stellt eine der häufigsten Todesursachen hospitalisierender Patienten dar. Vielfach wird als Ursache der Sepsis gramnegative Bakteriämie nachgewiesen.

Gleich dem septischen Schock zeigt auch die Shwartzman-Reaktion vergleichbare pathophysiologische Elemente. Die Shwartzman-Reaktion ist eine entzündlich, hämoorrhagisch und thrombotisch nekrotisierende Hautläsion, die durch lokale und anschließende intravenöse Endotoxingabe gramnegativer Bakterien ausgelöst wird. Der Shwartzman-Reaktion sind spezielle, im folgenden beschriebene Krankheitsbilder beim Menschen zuzuordnen.

Eine bei der Shwartzman-Reaktion makroskopisch auftretende Hautläsion ist etwa dem Syndrom der Purpura fulminans bei septischen Zuständen (z.B. Meningokokkensepsis) sehr ähnlich. Die Purpura fulminans ist ein lebensbedrohliches Krankheitsbild, das zu ausgedehnten Hautnekrosen und Autoamputationen der Extremitäten führt. Trotz herkömmlicher Therapieversuche, etwa mit Antibiotika oder intensivmedizinischen Maßnahmen ist die Mortalitätsrate sehr hoch.

Ebenso sind chronisch ulzerative Darmerkrankungen ein Beispiel für die Shwartzman-Reaktion beim Menschen. Für die Infektion der Darmwand, eine lokale, der Shwartzman-Reaktion entsprechende Läsion, sind ebenfalls zirkulierende Endotoxine auslösend.

Die Auswirkungen von septischem Schock, ausgelöst durch eine Infektion mit E. coli von Pavianen, werden in der Critical Care Medicine (1988) zusammengefaßt. Primär kommt es zu einer entzündlichen Reaktion und sekundär zu koagulopathischen Reaktionen und Zellschädigungen. Es wird erwähnt, daß die Infusion von aktiviertem Protein C vor den sekundären Folgen der Infektion schützt.

In der PCT-Anmeldung WO 90/08556 werden Kombinationspräparate zur Behandlung und Prophylaxe von Sepsis und septischem Schock beschrieben, welche einen bakterizid wirksamen Gehalt an Immunglobulin G sowie einen Blutgerinnsel-auflösenden Gehalt an aktiviertem Protein C aufweisen. Von der Verwendung vom Zymogen Protein C wird wegen möglicher Schwierigkeiten der in vivo Aktivierung abgeraten.

Gleichermaßen ist aus J. Clin. Invest. (1987) bekannt, daß bei Blockierung der in vivo Aktivierung von Protein C die Wirkung einer Infektion mit E. coli in Pavianen verschlimmert werden kann. Dies führt sogar bei Infektion mit subletalen Dosen an E. coli zum Tod der Versuchstiere. Der Tod kann jedoch durch Co-Infusion von aktiviertem Protein C verhindert werden. Eine präventive oder therapeutische Wirkung des Zymogens ist nicht ersichtlich.

Weiters ist bekannt, daß viele Patienten mit einem Mangel an Protein C das Purpura fulminans-Syndrom zeigen, das mit einem Arzneimittel enthaltend Protein C erfolgreich behandelt wird (Blood 10, Suppl. 1: 2070, 1990). Der Auslösemechanismus dieser Erkrankung unterscheidet sich jedoch wesentlich von einer durch eine mikrobielle Infektion ausgelösten Purpura fulminans. Eine gleichartige Behandlung ist deshalb im Fall einer Sepsis nicht naheliegend.

Ausgelöst durch Endotoxine kann es neben der Purpura fulminans auch zu einer Dermatitis ulcerosa kommen. Gegen diese Krankheitsbilder ist jedoch bislang kein Mittel zur Prophylaxe und Therapie bekannt.

Die Erfindung stellt sich die Aufgabe, das therapeutische Anwendungsgebiet von Protein C bzw. von aktiviertem Protein C zu erweitern.

Die Erfindung besteht in der Verwendung von Protein C bzw. aktiviertem Protein C zur Herstellung eines Arzneimittels zur Prophylaxe und Therapie der klinischen Äquivalente der lokalen und generalisierten Shwartzman-Reaktion, insbesondere mit der Maßgabe, daß das Arzneimittel kein Immunglobulin G enthält. Es hat sich gezeigt, daß sowohl Protein C als auch aktiviertes Protein C ohne Kombination mit bakteriziden Wirksubstanzen eine hervorragende Wirkung in den obengenannten Fällen zeigt.

Gleichfalls kann Protein C erfindungsgemäß zur Herstellung von Arzneimitteln zur Prophylaxe und Therapie von chronisch entzündlichen Darmerkrankungen und Prophylaxe und Therapie von Dermatitis ulcerosa verwendet werden.

Weitere bevorzugte Indikationen sind:
- Aufrechterhaltung und Verbesserung der Mikrozirkulation bei Patienten mit Durchblutungsstörungen;
- Niederregulierung von entzündlichen Prozessen, welche durch eine verringerte Mikrozirkulation bedingt sind und
- Prophylaxe und Therapie von Purpura fulminans, welche durch mikrobielle Infektion bedingt ist.

Protein C bzw. aktiviertes Protein C hat sich als besonders geeignet zur Behandlung von Durchblutungsstörungen bei malignen Erkrankungen, Autoimmunerkrankungen, Immunkomplex-Erkrankungen, Infektionserkrankungen und bei Schocksyndrom erwiesen.

Im Tiermodell konnte gezeigt werden, daß sowohl das Zymogen Protein C als auch aktiviertes Protein C die lokale Shwartzman-Reaktion abschwächt. Der Wirkungsmechanismus wird in der Hemmung der durch Endotoxin hervorgerufenen Leukozytenstimulation und Synthese und Freisetzung von Zytokinen vermutet. Weiters wird angenommen, daß die Endothelstimulation und Expression von Leukozytenadhäsionsmolekülen verhindert wird und die Leukozytenadhäsion unterdrückt wird. Es konnte nachgewiesen werden, daß Protein C bzw. aktiviertes Protein C die durch mikrobiologische Infektion ausgelöste Gerinnungsaktivierung und Thrombosierung in der Mikrozirkulation verhindert.

Die Gewinnung von Protein C und von aktiviertem Protein C, sowie die Prüfung auf Beeinflussung der lokalen Shwartzman-Reaktion werden nachfolgend beschrieben.

### Gewinnung von Protein C

Hochreines Protein C wurde aus einer rohen Protein C-Fraktion gewonnen, die aus kommerziell erhältlichem Prothrombinkomplex-Konzentrat hergestellt wurde. Die Reinigung erfolgte affinitätschromatographisch mittels monoklonaler Antikörper. Monoklonale Anti-Protein C-Antikörper wurden wie folgt hergestellt:

BALB/C-Mäuse wurden mit 100 µg menschlichem Protein C in zweiwöchigen Abständen durch intraperitoneale Injektion immunisiert. Nach sechs Wochen wurden noch einmal 50 µg des menschlichen Protein C injiziert und drei Tage später die Fusion vorgenommen. Die Myelomzellinie (P3-X-63-AG8-653, 1,5 x 10⁷ Zellen) wurde vermischt mit 1,7 x 10⁸ Milzzellen von einer Maus, und die Fusion nach modifizierter Köhler & Milestein-Methode unter Verwendung von PEG 1500 durchgeführt (Köhler G., Milestein C., Nature 256(1975)495-497).

Positive Klone, getestet mittels eines ELISA, wurden zweimal subgeklont. Aszitesproduktion erfolgte durch Injektion von 5 x 10⁶ Hybridomzellen je BALB/C-Maus zwei Wochen nach Pristan-Behandlung.

Das Immunglobulin wurde aus Aszites durch Ammoniumsulfatpräzipitation und anschließende Chromatographie mittels QAE-Sephadex (Pharmacia) und darauffolgend Chromatographie an Sephadex G200 (Pharmacia) gereinigt. Um das Risiko der Übertragung muriner Viren zu reduzieren, wurde der Antikörper vor der Immobilisierung noch einem Virusinaktivierungsschritt unterworfen. Die so erhaltenen monoklonalen Antikörper gegen Protein C wurden an CNBr-aktivierte Sepharose 4B (Pharmacia) gekoppelt. Für die Reinigung des Protein C mittels Affinitätschromatographie wurden folgende Puffer verwendet:

Als Adsorptionspuffer: 20 mM Tris, 2 mM EDTA, 0,25 M NaCl und 5 mM Benzamidin;
als Waschpuffer wurde verwendet: 20 mM Tris, 1 M NaCl, 2 mM Benzamidin, 2 mM EDTA; der pH-Wert betrug 7,4;
als Elutionspuffer wurde verwendet: 3 M NaSCN, 20 mM Tris, 1 M NaCl, 0,5 mM Benzamidin, 2 mM EDTA.

Im einzelnen: Das Prothrombinkomplex-Konzentrat wurde im Adsorptionspuffer aufgelöst, wobei etwa 10 g des Prothrombinkomplex-Konzentrates für eine 20 ml monoklonale Antikörpersäule zur Verwendung kamen. Anschließend wurde das aufgelöste Prothrombinkomplex-Konzentrat filtriert, bei 20.000 rpm 15 min lang zentrifugiert und durch ein 0,8 µm Filter sterilfiltriert. Das sterilfiltrierte und gelöste Prothrombinkomplex-Konzentrat wurde auf die Säule aufgetragen mit einer Flußrate von 10 ml/h. Anschließend wurde die Säule mit dem Waschpuffer proteinfrei gewaschen und schließlich das gebundene Protein C mit dem Elutionspuffer bei einer Flußrate von 5 ml/h eluiert und die Fraktionen gesammelt. Das eluierte Protein C wurde gegen einen Puffer (0,2 mol/l Tris, 0,15 M Glycin und 1 mM EDTA, pH 8,3) dialysiert. Der Protein C-Gehalt wurde antigenmäßig mittels der Methode nach Laurell und aktivitätsmäßig nach Protac-Aktivierung bestimmt.

Das erhaltene Protein C-Eluat wurde in folgender Weise zu einer pharmazeutisch applizierbaren Präparation fertiggestellt:

Das Eluat wurde zuerst einem Ultrafiltrations- und einem Diafiltrationsschritt unterworfen. Für die Diafiltration wurde ein Puffer mit einem pH-Wert von 7,4 verwendet, der pro Liter 150 mmol NaCl und 15 mmol Trinatriumcitrat.2H₂O enthielt. Das erhaltene Filtrat wurde gefriergetrocknet und durch eine einstündige Dampfbehandlung bei 80° C ± 5° C und 1375 ± 35 mbar virusinaktiviert.

Das lyophilisierte virusinaktivierte Material wurde sodann in einer sterilen isotonen NaCl-Lösung gelöst und mittels Ionenaustauschchromatographie an Q-Sepharose^{R} (Pharmacia) wurden etwaig vorhandene Antikörper bzw. Serumamyloid P entfernt. Die gereinigte Lösung wurde durch einen weiteren Ultrafiltrations- und Diafiltrationsschritt konzentriert. Danach wurden zur erhaltenen Lösung pro Liter 10 g Albumin, 150 mmol NaCl und 15 mmol Trinatriumcitrat zugegeben. Der pH-Wert der Lösung betrug 7,5. Maus-Immunglobulin sowie die Faktoren II, VII, IX und X konnten nicht nachgewiesen werden. Anschließend wurde die Lösung sterilfiltriert, abgefüllt und lyophilisiert. Die spezifische Aktivität betrug 14 Einheiten Protein C/mg. Eine Einheit Protein C Aktivität ist definiert als die Protein-C-Aktivität in 1 ml Normalplasma und wird gegen den ersten internationalen Standard von Protein C kalibriert. Als Aktivitätstest wurde ein amidolytischer Test verwendet, wobei das Protein C mittels Protac (Fa. Pentapharm) aktiviert wurde.

### Gewinnung von aktiviertem Protein C

Die Aktivierung des gereinigten Protein C erfolgte dadurch, daß 70 ml Thrombin (500 NIH Einheiten/ml, etwa 2000 NIH Einheiten/mg Protein entsprechend) an CNBr-aktivierte Sepharose 4B (Pharmacia) gekoppelt wurden, worauf Protein C mit dem Thrombingel in einem Verhältnis von etwa 6 Einheiten Protein C zu 1 Einheit Thrombin bei 37° C gemischt und unter kontinuierlichem Schütteln 3 h in Reaktion belassen wurden. Die Protein C-Aktivität wurde anschließend mit chromogenem Substrat (S 2366) bestimmt. Das aktivierte Protein C wurde anschließend sterilfiltriert und wie oben beschrieben zu einer pharmazeutischen Präparation fertiggestellt.

### Beeinflussung der lokalen Shwartzman-Reaktion am Kaninchen durch Protein C

### Methode:

Die Versuche wurden an Kaninchen (Weiße Neuseeländer) beiderlei Geschlechts im Gewicht von 2 bis 3 kg durchgeführt.

In Kurznarkose (20 mg/kg Ketamin^{R} + 5 mg/kg Rombun^{R} i.m.) wurde die Bauchhaut von Kaninchen mittels Rasur und Verwendung von Depilan^{R} von den Haaren befreit. Anschließend wurden jedem Tier intradermal 6 Quaddeln zu 0,2 ml Endotoxin von Salmonella typhimurium (Sigma L 7261) gesetzt. Die verwendeten Endotoxindosen betrugen 6,25; 12,5; 25; 50; 100 und 200 µg (präparative Dosen). 24 Stunden später erhielten die Kaninchen 20 µg/kg des Endotoxins i.v. in eine Ohrvene verabreicht (0,2 ml/kg; Provokationsdosis).

Es wurden geprüft:
A) aktiviertes Protein C (n = 5 Tiere) nach folgendem Schema intravenös: 250 E/kg unmittelbar nach der Provokationsdosis sowie 50 E/kg jeweils 1, 2 und 3 Stunden später. Das Injektionsvolumen betrug 0,25 ml/50 E.
B) Protein C (n = 7 Tiere), 500 E/kg i.v. unmittelbar nach der Provokationsdosis sowie 100 E/kg jeweils 1, 2 und 3 Stunden später. Das Injektionsvolumen betrug 0,8 ml/100 E.
C) Zu jeder Versuchsgruppe (A, B) wurde eine eigene Kontrollgruppe angelegt (n = 6 bzw. 5 Tiere). Diese Tiere erhielten neben den präparativen Endotoxindosen und der Provokationsdosis keine weitere Behandlung.

Die Beurteilung der Hautveränderungen erfolgte 6, 24 und 48 Stunden nach der Provokationsdosis. Beurteilt wurden die Parameter "Verdickung und Schwellung" (0 - 3), "Größe" (0 - 4), "Farbe" (0 - 4) und "Ringbildung" (0 - 1) (Die Zahlen in Klammer geben die Scores an, mit Hilfe derer jeder einzelne Parameter quantifiziert wurde). Die Scores der einzelnen Parameter wurden dann zu einem Gesamtscore addiert, durch das das Ausmaß der jeweiligen Hautveränderung charakterisiert wurde.

Die Gesamtscores je Dosisgruppe wurden sowohl für die Tiere der Versuchsgruppen als auch für die Kontrolltiere gemittelt und sind in der Tabelle dargestellt. Diese Ergebnisse stützen sich auf die 6 Stunden-Werte.

### Ergebnisse:

Es wurden Hautläsionen beobachtet, die von petechialer Rötung bis zu hochgradiger intradermaler Haemorrhagie mit oberflächlicher Krustenbildung reichten. Der Grad der Hautläsionen war von der Dosis der präparativen Toxininjektion und der Behandlung mit Testsubstanzen abhängig. Wie aus der Tabelle ersichtlich, sind die Veränderungen bei unbehandelten Kontrolltieren hochgradig, bei behandelten Kaninchen (aktiviertes Protein C, Protein C) lediglich mäßig ausgebildet.

**Tabelle**

| Shwartzman-Reaktion an Kaninchen | | | | | | | |
|---|---|---|---|---|---|---|---|
| Behandlungsgruppe | n | Hautläsionen, Score (Mittelwerte), an den Stellen präparativer Toxininjektion (µg) | | | | | |
| | | 6,25 | 12,5 | 25 | 50 | 100 | 200 |
| A | 5 | 0,4 | 0,2 | 2,8 | 4,6 | 5,0 | 5,8 |
| C_{A} | 6 | 2,5 | 4,7 | 5,4 | 8,3 | 8,7 | 9,7 |
| B | 7 | 1,0 | 1,1 | 2,0 | 3,3 | 3,6 | 5,4 |
| C_{B} | 5 | 1,2 | 2,2 | 4,8 | 7,0 | 7,8 | 8,4 |

Aktiviertes Protein C (400 E/kg i.v.) und Protein C (800 E/kg i.v.) sind hinsichtlich des dargestellten protektiven Effektes vergleichbar. Trägt man die Mittelwerte der Scores (siehe Tabelle) gegen die Dosis in logarithmischem Maßstab auf, so resultieren parallele Dosiswirkungskurven.

Vorbehandlung mit aktiviertem Protein C und Protein C verschiebt die für die entsprechenden Kontrollen erstellten Dosiswirkungskurven jeweils um den Faktor 4 nach rechts, zu höheren Dosen hin.

## Patentansprüche

1. Verwendung von Protein C bzw. aktiviertem Protein C zur Herstellung eines Arzneimittels zur Prophylaxe und Therapie der klinischen Äquivalente der lokalen und generalisierten Shwartzman-Reaktion, insbesondere mit der Maßgabe, daß das Arzneimittel frei von Immunglobulin G ist.

2. Verwendung nach Anspruch 1 zur Prophylaxe und Therapie von chronisch entzündlichen Darmerkrankungen.

3. Verwendung nach Anspruch 1 zur Prophylaxe und Therapie von Dermatitis ulcerosa.

4. Verwendung nach Anspruch 1 zur Aufrechterhaltung und Verbesserung der Mikrozirkulation bei Patienten mit Durchblutungsstörungen.

5. Verwendung nach Anspruch 4, wobei die Durchblutungsstörungen bei malignen Erkrankungen, Autoimmunerkrankungen, Immunkomplex-Erkrankungen, Infektionserkrankungen und bei Schocksyndrom auftreten.

6. Verwendung nach einem der Ansprüche 1 bis 5 zur Niederregulierung von entzündlichen Prozessen, welche durch eine verringerte Mikrozirkulation bedingt sind.

7. Verwendung nach einem der Ansprüche 1 bis 6 zur Prophylaxe und Therapie von Purpura fulminans, welche durch mikrobielle Infektion bedingt ist.

## Claims

1. The use of protein C or activated protein C, respectively, for producing a medicament for the prevention and therapy of the clinical equivalents of the local and generalized Shwartzman reaction, in particular with the proviso that the medicament is free from immunoglobulin G.

2. The use according to claim 1 for the prevention and therapy of chronic inflammatory diseases of the intestines.

3. The use according to claim 1 for the prevention and therapy of dermatitis ulcerosa.

4. The use according to claim 1 for maintaining and improving the microcirculation in patients suffering from circulatory disturbances.

5. The use according to claim 4, wherein the circulatory disturbances are associated with malign diseases, autoimmune diseases, immunocomplex diseases, infectious diseases and with shock syndrome.

6. The use according to any one of claims 1 to 5, for down-regulation of inflammatory processes caused by a reduced microcirculation.

7. The use according to any one of claims 1 to 6 for the prevention and therapy of purpura fulminans caused by microbial infection.

## Revendications

1. Utilisation de protéine C ou de protéine C activée pour la préparation d'un médicament pour la prévention et le traitement des équivalents cliniques du phénomène local et généralisé de Shwartzman-Saranelli, notamment à la condition que le médicament soit exempt d'immunoglobuline G.

2. Utilisation selon la revendication 1 pour la prévention et le traitement d'entéropathies inflammatoires chroniques.

3. Utilisation selon la revendication 1 pour la prévention et le traitement de la dermatite ulcéreuse.

4. Utilisation selon la revendication 1 pour le maintien et l'amélioration de la microcirculation chez des malades présentant des troubles circulatoires.

5. Utilisation selon la revendication 4, les troubles circulatoires se manifestant au cours d'affections malignes, de maladies auto-immunes, de maladies des complexes immuns, de maladies infectieuses ou lors du syndrome du choc.

6. Utilisation selon une des revendications 1 à 5 pour l'atténuation de processus inflammatoires dus à un ralentissement de la microcirculation.

7. Utilisation selon une des revendications 1 à 6 pour la prévention et le traitement du purpura fulminans dû à une infection microbienne.
